# EUROPÄISCHE PATENTSCHRIFT

(11) **EP 2 382 181 B1**
(45) Veröffentlichungstag und Bekanntmachung des Hinweises auf die Patenterteilung: **30.04.2014**
(21) Anmeldenummer: 09799345.5
(22) Anmeldetag: 21.12.2009
(51) Int. Cl.: C07C 209/36, C07C 211/50

(54) **VERFAHREN ZUR HERSTELLUNG VON TOLUYLENDIAMIN DURCH HYDRIERUNG VON DINITROTOLUOL**
METHOD FOR PRODUCING TOLUYLENEDIAMINE BY HYDROGENATING DINITROTOLUENE
PROCÉDÉ DE PRODUCTION DE TOLUYLÈNE DIAMINE PAR HYDROGÉNATION DE DINITROTOLUÈNE

(30) Priorität: 29.12.2008 DE 102008063308
(43) Veröffentlichungstag der Anmeldung: 02.11.2011
(73) Patentinhaber: BASF SE, 67056 Ludwigshafen (DE)
(72) Erfinder: COELHO TSOU, Joana, 69120 Heidelberg (DE); OEHLENSCHLÄGER, Steffen, 2050 Antwerpen (BE); SCHWAB, Ekkehard, 67434 Neustadt (DE); MACKENROTH, Wolfgang, B-3080 Tervuren (BE); VOß, Hartwig, 67227 Frankenthal (DE); MAIXNER, Stefan, 68723 Schwetzingen (DE); NETO, Samuel, 01099 Dresden (DE); BÖHMEKE, Sven, 01968 Senftenberg (DE); VAN LAAR, Frederik, 3604 Maarsen (NL)
(86) Internationale Anmeldenummer: PCT/EP2009/067610
(87) Internationale Veröffentlichungsnummer: WO 2010/076251

(56) Entgegenhaltungen:
- EP-A1- 0 124 010
- EP-A1- 0 223 035
- EP-A1- 0 634 391
- WO-A1-00/35852

## Beschreibung

Die Erfindung betrifft ein Verfahren zur Herstellung von Toluylendiamin durch Hydrierung von Dinitrotoluol.

Toluylendiamin ist ein in der Technik häufig eingesetztes aromatisches Amin; es wird insbesondere zu Toluylendiisocyanat weiterverarbeitet, das überwiegend in der Polyurethanherstellung Verwendung findet. Toluylendiamin wird durch katalytische Hydrierung von Dinitrotoluol hergestellt.

Es wurden eine Vielzahl von Katalysatoren für die obige Umsetzung entwickelt, um eine möglichst hohe Ausbeute und Selektivität in der Umsetzung zu erreichen und darüber hinaus Katalysatoren aufzufinden, die auch bei höheren Reaktionstemperaturen stabil sind.

Bei der Hydrierung von Dinitrotoluol wird eine große Menge an Reaktionswärme frei. Daher war es schon immer das Bestreben, diese Reaktionswärme zum Beispiel in Form von Dampf zu nutzen.

Dokument US 5,563,296 beschreibt ein Verfahren und einen Reaktor zur Herstellung von Toluylendiamin durch Hydrierung von Dinitrotoluol in einem Reaktor mit externer Schlaufe, wobei die Wärmeabführung ausschließlich in der externen Schlaufe erfolgt. Nachteilig an diesem Verfahren ist, dass bei der Erzeugung von Dampf die Siedetemperatur des Dampfes signifikant unterhalb der Reaktionstemperatur liegen muss, da ansonsten die Fläche des externen Wärmeübertragers und die umzuwälzende Flüssigkeitsmenge zu groß für ein wirtschaftliches Verfahren werden.

Die Hydrierung von Dinitrotoluol ist stark exotherm. Als ein für Abführung der Reaktionswärme besonders geeigneter Reaktor wurde daher in WO 00/35852 ein Reaktor mit interner und externer Schlaufenbewegung vorgeschlagen, der als vertikal aufrecht stehender Apparat ausgebildet ist, mit einer Treibstrahldüse an seinem oberen Ende, über die in den oberen Bereich des Reaktors das aus dem Reaktorsumpf abgezogene Reaktionsgemisch über eine externe Schlaufe eingedüst wird, und das anschließend in ein zentrales Einsteckrohr, welches in Reaktorlängsrichtung angeordnet ist, einströmt, dieses von oben nach unten durchströmt und in einer internen Schlaufenbewegung außerhalb des Einsteckrohrs erneut nach oben strömt. Für die Abführung der Reaktionswärme sind im Reaktorinnenraum Wärmetauscher, insbesondere Fieldrohre vorgesehen, das heißt vertikal in Reaktorlängsrichtung angeordnete Doppelrohre, wovon das innere Rohr unten offen und das äußere Rohr unten geschlossen ist und in denen ein Wärmeträger, insbesondere Wasser strömt, und die Reaktionswärme abführt.. Zusätzlich zur Wärmeabführung über im Reaktorinnenraum angeordnete Wärmetauscher kann auch ein Wärmetauscher in der externen Schlaufenströmung vorgesehen sein.

Die EP-A 124010 beschreibt eine Blasensäule zur Hydrierung von Dinitrotoluolverbindungen zu den entsprechenden Aminen. Die Reaktionswärme wird über Fieldrohre abgeführt. Ziel ist es, Dampf mit einem Überdruck von > 1 bar zu erzeugen. Es wird hierfür eine auf das Reaktionsvolumen bezogene spezifische Wärmeübertragerfläche von 40 bis 400 m²/m³ zu Grunde gelegt. Der Nachteil dieser Anordnung ist, dass bei derart großen Kühlflächen der Abstand zwischen den Kühlrohren so gering wird, dass mit Fouling und Feststoffablagerungen zu rechnen ist. Darüber hinaus wird die makroskopische Durchmischung des Apparates massiv gestört.

Die EP-A 0 223 035 beschreibt ein gattungsgemäßes Verfahren zur Herstellung von Toluylendiamin durch Hydrierung von Dinitrotuluol, wobei durch Einsatz von mittels einer Alkalibehandlung modifizierten Raney-Katalysatoren eine Verfahrensführung ermöglicht wird, die es gestattet, die Reaktionswärme der Hydrierung als Dampf zu nutzen, insbesondere mit Abgabedrucken im Bereich zwischen 5 und 30 bar, ohne Einbußen in Produktqualität und Ausbeute. Das Verfahren ist nicht eingeschränkt auf den Einsatz spezieller Reaktoren, es kann, im Gegenteil, unter Verwendung üblicher Reaktoren erfolgen.

Die Herausforderung bei allen obigen Verfahren ist, dass sich eine niedrige Reaktionstemperatur, das heißt eine geringe Differenz zwischen Reaktionstemperatur und Dampftemperatur, zwar positiv auf die Selektivität der Reaktion auswirkt, jedoch zu unwirtschaftlich großen Wärmeübertragerflächen führt.

Es war daher Aufgabe der Erfindung, ein wirtschaftlich attraktives Verfahren zur katalytischen Hydrierung von Dinitrotoluol zu Toluylendiamin zu finden, bei welchem das Temperaturniveau des Dampfes möglichst nahe bei der Reaktionstemperatur liegt. Dabei soll Dampf mit einem Überdruck ≥ 4 bar erzeugt werden.

Die Lösung besteht in einem Verfahren zur Herstellung von Toluylendiamin durch Hydrierung von Dinitrotoluol mit Wasserstoff in Gegenwart eines suspendierten Katalysators
- in einem vertikal aufrechtstehenden Reaktor,
- an dessen oberem Ende eine Treibstrahldüse angeordnet ist, über die in den oberen Bereich des Reaktors das aus dem Reaktorsumpf abgezogene Reaktionsgemisch über eine externe Schlaufe eingedüst wird, das anschließend in ein zentrales Einsteckrohr, welches in Reaktorlängsrichtung angeordnet ist, einströmt, dieses von oben nach unten durchströmt und in einer internen Schlaufenbewegung außerhalb des Einsteckrohrs erneut nach oben strömt,
- mit einem Wärmetauscher im Innenraum des Reaktors, durch den Kühlwasser strömt, und hierbei einen Teil der Reaktionswärme aufnimmt,
- mit Zuführung des Dinitrotoluols am oberen Endes des Reaktors und Zuführung des Wasserstoffs am unteren Ende des Reaktors,
- und wobei zusätzlich zu dem im Innenraum des Reaktors angeordneten Wärmetauscher ein weiterer Wärmetauscher, in der externen Schlaufe eingesetzt wird, worin Wasser durch indirekten Wärmetausch mit der Reaktionsmischung die restliche Reaktionswärme aufnimmt,
das dadurch gekennzeichnet ist, dass
die Reaktionswärme zur Gewinnung von Dampf mit einem Überdruck von mindestens 4 bar genutzt wird, indem die Hydrierung von Dinitrotoluol zu Toluylendiamin bei einer Temperatur ≥180°C durchgeführt wird.

Bevorzugt wird die Hydrierung von Dinitrotoluol zu Toluylendiamin bei einer Temperatur ≥ 185°C durchgeführt.

Es wurde gefunden, dass es möglich ist, die Hydrierung von Dinitrotoluol zu Toluylendiamin dergestalt durchzuführen, dass nicht nur eine hohe Selektivität und Ausbeute der Reaktion selbst erreicht wird, sondern dass darüber hinaus die Reaktionswärme genutzt werden kann, um Dampf auf einem hohen Druckniveau, von 4 bar Überdruck oder höher, zu erzeugen, wie er üblicherweise in den Dampfleitungen in industriellen Anlagen zur Verfügung gestellt wird. Dadurch ist es möglich, den Dampf in das vorhandene Dampfnetz einzuspeisen und/oder in der Gesamtanlage, ausgehend von Toluol über Dinitrotoluol, Toluylendiamin bis zur Herstellung von Toluylendiisocyanat, zu nutzen.

Alternativ ist es auch möglich, Dampf auf zwei Druckniveaus von 4 bar Überdruck, oder höher, zur Verfügung zu stellen.

Für die Hydrierung von Dinitrotoluol zu Toluylendiamin wurden eine Vielzahl von Katalysatoren entwickelt, wobei die Verbesserung von Ausbeute und Selektivität der Umsetzung sowie die Stabilität der Katalysatoren auch bei höheren Reaktionstemperaturen vorrangige Aufgaben bei der Entwicklung neuer Katalysatoren waren.

Als besonders geeignet haben sich Katalysatoren erwiesen, die als Aktivmasse ein oder mehrere Metalle, ausgewählt aus der Gruppe Platin, Palladium, Rhodium und Ruthenium und daneben ein oder mehrere weitere Metalle, ausgewählt aus der Gruppe Nickel, Kobalt, Eisen und Zink enthalten, und die auf einem inerten Träger aufgebracht ist.

Vorteilhaft sind insbesondere Katalysatoren, die als Aktivmasse Platin und daneben Nickel, oder auch Katalysatoren, die Palladium, Nickel und Eisen oder Katalysatoren, die Palladium, Nickel und Kobalt, enthalten.

Besonders vorteilhaft sind Hydrierkatalysatoren, die Platin und Nickel auf einem Träger in Form einer Legierung mit einem Atomverhältnis von Nickel zu Platin in der Legierung zwischen 30 : 70 und 70 : 30, enthalten.

Legierungen aus Platin und Nickel mit anderen Atomverhältnissen sind für das erfindungsgemäße Verfahren prinzipiell auch brauchbar, sie führen jedoch, insbesondere bei der Durchführung der Hydrierung bei höheren Temperaturen, zu geringen Ausbeuten an TDA.

Das Atomverhältnis von Nickel zu Platin, bestimmt mittels EDXS (Energy Dispersive X-Ray Spectroscopy), liegt insbesondere bei zwischen 45 : 55 und 55 : 45.

Der Katalysator enthält zumeist ca. 1 bis 15 nm große, feinkristalline Metallpartikel der Pt-Ni Legierung verteilt auf z.B. Kohlenstoffteilchen. Stellenweise können 1- bis 2-µm große Ni-Pt Teilchenagglomerate oder -aggregate, aber auch vereinzelte reine Ni oder Pt Teilchen auf dem Träger vorkommen. Die Elektronenbeugungslinien der Metallteilchen liegen zwischen denen von Pt und Ni, welche die Legierungsbildung zusätzlich belegt. Die Metallpartikel sind meist polykristallin, und können mit einem hoch-auflösenden TEM (FEG-TEM: Field Emission Gun-Transmission Electron Microscopy) charakterisiert werden.

Als Träger für die Katalysatoren können die hierfür üblichen und bekannten Materialien eingesetzt werden. Vorzugsweise werden Aktivkohle, Ruß, Graphit oder Metalloxide, bevorzugt hydrothermal stabile Metalloxide, wie z.B. ZrO₂, TiO₂ eingesetzt. Bei Graphit sind die HSAG (High Surface Area Graphite) mit einer Oberfläche von 50 bis 300 m²/g besonders bevorzugt. Besonders bevorzugt sind die physisch oder chemisch aktivierte Aktivkohle oder Ruße, wie Acetylenruß.

Ziel des Verfahrens ist es, Dampf auf einem Temperaturniveau ≥ 4 bar Überdruck zur Verfügung zu stellen. Dabei soll die Reaktionstemperatur nicht zu hoch gewählt werden, um Selektivitätsverluste durch Nebenreaktionen (zum Beispiel Hochsiederbildung) zu vermieden. Dies führt zu großen erforderlichen Wärmeübertragerflächen. Wird der Wärmeübertrager zur Abführung der Reaktionswärme ausschließlich in den Reaktor integriert, resultiert das in einem sehr großen Reaktorvolumen und daher in sehr geringen Raum-Zeit-Ausbeuten. Wird der Wärmeübertrager ausschließlich in den externen Kreislauf integriert, resultiert das in einem sehr großen Kreislaufstrom, da das Medium im externen Wärmeübertrager nicht zu stark abgekühlt werden kann, um Dampf auf hohem Druckniveau zur Verfügung zu stellen.

Daher kann der Dampf nur wirtschaftlich zur Verfügung gestellt werden, wenn die Wärmeabfuhr gleichzeitig in dem Apparat und in dem externen Kreislauf erfolgt. Dies führt gleichzeitig zu akzeptablen Reaktorvolumina und großen Raum-Zeit-Ausbeuten.

Das Verfahren wird daher bevorzugt bei Raum-Zeit-Ausbeuten von größer 150 kg Toluylendiamin pro m³ begastes Reaktorvolumen und Stunde, insbesondere bei Raum-Zeit-Ausbeuten von größer 250 kg Toluylendiamin per m³ begastes Reaktorvolumen und Stunde durchgeführt.

Das Verfahren wird bevorzugt ohne Zusatz eines Lösungsmittels durchgeführt.

Das erfindungsgemäße Verfahren wird in einem Reaktor durchgeführt, in dem eine interne und eine äußere Schlaufenbewegung des Reaktionsgemisches stattfindet.

Hierzu ist in einem vertikal aufrecht stehenden Reaktor an seinem oberen Ende eine Treibstrahldüse angeordnet, die die innere Schlaufenbewegung antreibt, und zwar indem über die Treibstrahldüse in den oberen Bereich des Reaktors das aus dem Reaktorsumpf abgezogene Reaktionsgemisch, das über eine externe Schlaufe umgepumpt wird, in den oberen Bereich des Reaktors nach unten gerichtet eingedüst wird. Das über die Treibstrahldüse eingedüste Reaktionsgemisch strömt durch ein zentrales, in Reaktorlängsrichtung angeordnetes Einsteckrohr und durchströmt dieses von oben nach unten. Das zentrale Einsteckrohr kann als einfaches Rohr ausgestaltet sein. Unterhalb des Einsteckrohrs kehrt sich das Reaktionsgemisch in einer internen Schlaufenbewegung um und strömt erneut nach oben. Zur Strömungsumkehr ist bevorzugt eine Prallplatte unterhalb des Einsteckrohrs angeordnet.

Durch das konzentrische Einsteckrohr wird in Verbindung mit der Prallplatte die Schlaufenströmung innerhalb des Reaktors, das heißt die interne Schlaufenströmung, stabilisiert.

Durch den Treibstrahl wird Gas aus dem Gasraum in die Flüssigkeit in Form von Gasblasen bis zur Prallplatte mitgerissen. Diese Gasblasen steigen im Ringraum des Reaktors wieder auf. Durch diese interne Kreisgasfahrweise wird eine große Gas/Flüssig-Phasengrenzfläche zur Verfügung gestellt.

Im Reaktorinnenraum ist zur Wärmeabführung ein Wärmetauscher angeordnet, durch den Kühlwasser strömt, das einen Teil der Reaktionswärme aufnimmt.

Der im Reaktorinnenraum angeordnete Wärmetauscher ist bevorzugt ein Fieldrohr-Wärmetauscher.

In einer Ausführungsform ist der im Innenraum des Reaktors angeordnete Wärmetauscher ein Rohrschlangen-Wärmetauscher.

In einer weiteren Ausführungsform ist der im Innenraum des Reaktors angeordnete Wärmetauscher ein Rohrbündel-Wärmetauscher.

Der restliche Teil der Reaktionswärme wird über einen Wärmeübertrager abgeführt, der in der externen Schlaufe angeordnet ist. Bevorzugt kommt dabei ein Rohrbündel-Wärmeübertrager zum Einsatz.

Aus der freiwerdenden Reaktionswärme kann Dampf in sowohl in dem internen als auch dem externen Wärmeübertrager auf zwei Arten erzeugt werden: 1) Durch Verdampfung eines Teils des Kühlwassers in den Kühlrohren (Direktdampferzeugung) oder 2) durch Erhitzen des Kühlwassers auf einen Druck, der oberhalb des Druckes des zu erzeugenden Dampfes liegt, und anschließende Entspannung auf das Druckniveau des zu erzeugenden Dampfes (Entspannungsverdampfung). Bei dieser Entspannung verdampft ein Teil des Kühlwassers, und das Dampf/Wasser-Gemisch kühlt sich auf die dem Druck des Dampfes entsprechende Siedetemperatur ab.

Beide Arten der Verdampfung können sowohl im internen und als auch externen Wärmeübertrager angewendet werden. Ebenso ist eine Kombination beider Verdampfungsarten möglich, das heißt Direktverdampfung im internen Wärmeübertrager und Entspannungsverdampfung im externen Wärmeübertrager oder umgekehrt.

Dinitrotoluol wird am oberen Ende des Reaktors zugeführt, bevorzugt in die Gasphase oberhalb des Flüssigkeitsspiegels im Reaktor.

Vorteilhaft wird das Dinitrotoluol zur Vergleichmäßigung der Zuführung über einen Ringverteiler zugeführt.

In einer weiteren Ausführungsform ist die Treibstrahldüse, die im oberen Bereich des Reaktors angeordnet ist, als Zweistrahldüse ausgebildet, und das Dinitrotoluol wird über einen Ringspalt an der Außenwand der als Zweistrahldüse ausgebildeten Treibstrahldüse in den Reaktor geleitet.

Die Erfinder haben erkannt, dass es vorteilhaft ist, das Dinitrotoluol in die Gasphase oberhalb des Flüssigkeitsspiegels im Reaktor einzuleiten; hierdurch wird verhindert, dass Reaktionsprodukt zurück in die Zuführleitung für Dinitrotoluol strömt und das es dadurch zur Zersetzung oder Explosion des Dinitrotoluols führt. Reines Dinitrotoluol hat eine Zersetzungstemperatur von etwa 260°C, die Zersetzungstemperatur sinkt jedoch drastisch sobald Toluylendiamin zugemischt wird, bis auf 100°C.

Daher ist es auch vorteilhaft, die Zuführleitung des Dinitrotoluols bei Unterbrechungen der Produktion oder Abstellungen der Anlage mit heißem Wasser frei zu spülen.

Die Erfinder haben erkannt, dass es wesentlich ist, dass sich im Reaktor weniger als 1000 ppm Dinitrotoluol akkumuliert.

Der für die Hydrierung notwendige Wasserstoff wird am unteren Ende des Reaktors, bevorzugt über einen Ringverteiler, zugeführt.

Die Erfinder haben erkannt, dass es vorteilhaft ist, durch geeignete Maßnahmen sicherzustellen, dass die Konzentration des Wasserstoffs im Reaktionsgemisch, das aus dem Reaktorsumpf in die externe Schlaufe strömt, 1 Vol.-%, bevorzugt 3 Vol.-%, bezogen auf das Gesamtvolumen des Reaktionsgemisches, das in der externen Schlaufe strömt, nicht unterschreitet. Hierzu ist es möglich, den Durchmesser des Reaktors oder die Abströmgeschwindigkeit des Reaktionsgemisches aus dem Reaktor entsprechend auszulegen.

In einer weiteren Ausführungsform ist es möglich, die Mindestkonzentration des Wasserstoffs, der aus dem Reaktorsumpf in die externe Schlaufe strömt sicherzustellen, indem in das Reaktionsgemisch, das aus dem Reaktorsumpf in die externe Schlaufe strömt, möglichst nahe am Reaktor Wasserstoff zugeführt wird.

Um den externen Wälzkreis antreiben zu können, muss eine Pumpe installiert werden, die neben Flüssigkeit auch Gas und suspendierten Feststoff fördern kann. Dabei sollten bis zu 20 Vol.-% Gas und 20 Gew.-% suspendierter Feststoff pumpbar sein.

Der erzeugte Dampf kann in ein bestehendes Dampfnetz des Standortes der Anlage eingespeist werden, oder direkt in der Gesamtanlage, ausgehend vom Toluol über Dinitrotoluol, Toluylendiamin bis zur Herstellung von Toluylendiisocyanat genutzt werden.

Der durch das erfindungsgemäße Verfahren erzeugte Dampf auf einem Niveau von mindestens 4 bar Überdruck kann an vielen Stellen innerhalb und außerhalb des Toluylendiisocyanat (TDI)-Prozesses eingesetzt werden. In der Folge sind einige Einsatzmöglichkeiten aufgeführt, was jedoch nicht bedeuten soll, dass sich der Einsatz des erzeugten Dampfes nur auf diese Möglichkeiten beschränkt.

Der Dampf kann innerhalb des gesamten TDI-Komplexes, in dem die Toluylendiamin (TDA)-Stufe nur einen Teil darstellt, zur Wärme- oder Energiezufuhr verwertet werden. Insbesondere eignet er sich zum Einsatz in einer oder mehrerer der nachfolgend aufgeführten Verfahrensstufen:
- bei der Verdampfung bzw. Destillation zur Aufkonzentrierung von Säuren auf der Nitrier-(Dinitrotoluol-) Stufe,
- bei der Wasserabtrennung im Verdampfer der TDA/Wasser-Destillation,
- bei der Abtrennung von TDA von hochsiedendem Rückstand (Destillation oder Verdampfung),
- in der TDI-Synthesestufe bei der Phosgen- oder HCI-Strippung,
- bei der Beheizung des Reaktors oder der Reaktionskolonne zur Phosgenierung von TDA,
- im Verdampfer bei der TDI-Destillation,
- bei der Abdampfung des in der Phosgenierung verwendeten Lösungsmittels,
- und beim Betrieb von Vakuumjets (Erzeugung von Vakuum durch Entspannung des Dampfes) im gesamten TDI-Komplex.

Darüber hinaus kann der Dampf in einem oder mehreren der nachfolgend aufgeführten Bereiche eingesetzt werden:
- zum Abdampfen von Lösungsmitteln aus Prozessabwasser,
- zur Erzeugung von Kälte durch Absorptionskältemaschinen oder Dampfstrahlkälteanlagen,
- zur Erzeugung von Vakuum bei der Entspannung in Ejektoren,
- zur Erzeugung von Strom als Energie- oder Wärme-Quelle für Wärmekraftprozesse (Direktentspannung in Turbine oder ORC- bzw. Kalina-Kreisläufe),
- zur Beheizung von Rohrleitungen, Apparaten, Behältern und Tanks,
- und zur Beheizung von Gebäuden.

Darüber hinaus kann der Dampf auch in ein vorhandenes Dampfnetz am Standort der Anlage eingespeist werden.

Die Erfindung wird im Folgenden anhand eines Ausführungsbeispiels sowie einer Figur näher erläutert.

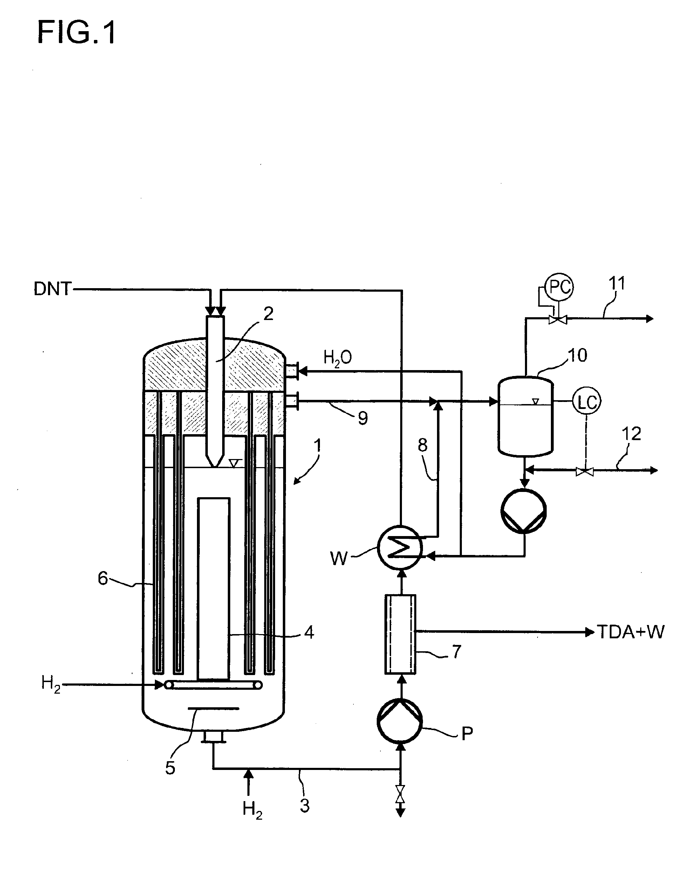

Die einzige Figur 1 zeigt die schematische Darstellung einer Anlage zur Durchführung des erfindungsgemäßen Verfahrens.

Der Reaktor 1 ist mit einer in seinem oberen Bereich, nach unten ausgerichteten Treibstrahldüse 2 ausgestattet, über die das Reaktionsgemisch über eine externe Schlaufe 3 in den Reaktor eingedüst wird. Unterhalb der Treibstrahldüse 2 ist ein zentraler, in Reaktorlängsrichtung angeordnetes Einsteckrohr 4 angeordnet und unterhalb des Einsteckrohrs 4 eine Prallplatte 5. Im Innenraum des Reaktors 1 befindet sich ein Fieldrohr-Wärmetauscher 6. Dinitrotoluol, DNT, wird in der in der Figur dargestellten bevorzugten Ausführungsvariante über einen Ringspalt an der Außenwand der als Zweistrahldüse ausgebildeten Treibstrahldüse 2 in den Gasraum oberhalb des Flüssigkeitsspiegels in den Reaktor 1 zugeführt. Wasserstoff H₂, wird in den unteren Bereich des Reaktors 1, in der in der Figur dargestellten bevorzugten Ausführungsvariante, über einen Ringverteiler 7, und zusätzlich in die externe Schlaufe 3, in der Nähe des Abzugs des Reaktionsgemisches aus dem Reaktorsumpf, eingedüst. Das Reaktionsgemisch wird in der externen Schlaufe über eine Pumpe P, die so ausgelegt ist, dass sie bis zu 20 Vol.-% Gas fördern kann, in der in der Figur dargestellten bevorzugten Ausführungsform über einen Querstromfilter 7 zur Katalysatorabtrennung geleitet. Das Reaktionsgemisch wird anschließend über einen in der externen Schlaufe angeordneten Wärmetauscher W geleitet, der bevorzugt als Rohrbündelwärmetauscher ausgebildet ist. Aus dem in der externen Schlaufe angeordneten Wärmetauscher W wird Dampf über Leitung 8 abgezogen und mit Dampf über Leitung 9 aus den Fieldrohren, die mit Wasser, H₂O, gespeist werden, vereinigt, einem Abscheider 10 zugeführt und als Dampf mit 4 bar Überdruck über Leitung 11 abgezogen. Über Leitung 12 wird ein Teilstrom des Kondensats abgezogen.

### Ausführungsbeispiel:

In einer Anlage, wie schematisch in Figur 1 dargestellt, wurde Dinitrotoluol zu Toluylendiamin in einem Reaktor mit externer Schlaufe 3 mit 170 l Flüssigvolumen (Gesamtvolumen Reaktor 1 + externe Schlaufe 3) bei 180°C und 25 bar Überdruck hydriert. In der externen Schlaufe 3 wurden 4,5 m³/h Flüssigkeit mit einer Pumpe P, die auch vorhandene Gasblasen und suspendierte Feststoffpartikel pumpen kann, umgewälzt. Im Reaktor 1 wurde ein Gasgehalt von ca. 15 Vol.-% eingestellt. Das Hydrierbad enthielt 2 Gew.-% eines suspendierten Katalysators mit 3 Gew.-% Pt und 1 Gew.-% Ni auf Kohlenstoffträger. Zur Katalysatorrückhaltung wurde ein Querstromfilter 7 mit einer Membran mit 1000 nm Porenweite eingesetzt. Am unteren Ausgang des Reaktors 1 wurde eine geringe Menge Wasserstoff in die äußere Schlaufe 3 eingespeist.

Es wurde ein Feedstrom von 62 kg/h DNT zugefahren. Dieses DNT wurde im Reaktor 1 vollständig umgesetzt. Damit ergab sich eine Raum-Zeit-Ausbeute von 245 kg TDA/(m³/h). Die Selektivität zu TDA war 98,69%, diejenige zu Schwersiedern 1,25% und diejenige zu Leichtsiedern 0,06%. Im Reaktor 1 wurde eine Reaktionswärme von 106 kW freigesetzt.

Soll nun Dampf auf einem Druckniveau von 4 bar Überdruck und bei einer Temperatur von 155°C erzeugt werden, so wird erfindungsgemäß ein Teil der Reaktionswärme im Reaktor 1 unter nahezu isothermen Bedingungen und der restliche Teil der Reaktionswärme in der externen Schlaufe 4 abgeführt.
Für die Wärmeabfuhr im Inneren des Reaktors 1 ist ein Fieldrohr-Wärmetauscher 6 im Ringraum um das Einsteckrohr 4 vorgesehen, und es wird eine spezifische Wärmetauscherfläche von 15 m²/m³ angenommen. Dadurch wird nur ein kleiner Teil der Querschnittsfläche des Reaktors 1 blockiert und die Strömung im Reaktorinneren nicht zu stark behindert. Die Wärmetauscherfläche des Fieldrohr-Wärmetauschers 6 beträgt damit 2,55 m² und es werden bei einem Wärmedurchgangskoeffizient von 1100 W/(m²/K) insgesamt 70 kW Reaktionswärme abgeführt.
Die restlichen 36 kW Reaktionswärme werden über den externen Wärmetauscher W im Kreislauf abgeführt. Bei einer spezifischen Wärmekapazität des Hydrierbades von 3,3 kJ/(kg/K) kühlt sich die Flüssigkeit dabei von 180°C vor dem externen Wärmetauscher W auf 171 °C nach dem externen Wärmetauscher W ab. Zur Wärmeabfuhr steht eine mittlere logarithmische Temperaturdifferenz von 20 K zur Verfügung. Wird ein Wärmedurchgangskoeffizient von 1000 W/(m²/K) zu Grunde gelegt, wird eine Wärmeübertragerfläche von 1,80 m² benötigt. Als externen Wärmetauscher 6 kommt dabei ein Rohrbündelwärmetauscher zum Einsatz.

Würde zum Vergleich die gesamte Reaktionswärme im Inneren des Reaktors 1 abgeführt, so wäre eine spezifische Wärmeübertragerfläche von 23 m²/m³ (d.h. 3,9 m² absolut) erforderlich. Hierbei würde ein großer Teil der Querschnittsfläche des Reaktors 1 mit Wärmetauscherrohren blockiert, und die Gefahr von Fouling im Reaktorinneren aufgrund von zu stark abgebremster Strömung wäre groß.
Wird stattdessen die gesamte Reaktionswärme mittels des externen Wärmetauschers W abgeführt, so könnte bei dem vorgegebenen Wälzstrom von 4,5 m³/h nicht mehr Dampf auf einem Temperaturniveau von 155°C erzeugt werden, da in diesem Fall die Temperatur des umgepumpten Hydrierbades am Ausgang des externen Wärmetauschers unterhalb von 155°C liegen würde.

## Patentansprüche

1. Verfahren zur Herstellung von Toluylendiamin durch Hydrierung von Dinitrotoluol mit Wasserstoff in Gegenwart eines suspendierten Katalysators
- in einem vertikal aufrechtstehenden Reaktor (1),
- an dessen oberem Ende eine Treibstrahldüse (2) angeordnet ist, über die in den oberen Bereich des Reaktors (1) das aus dem Reaktorsumpf abgezogene Reaktionsgemisch über eine externe Schlaufe (3) eingedüst wird, das anschließend in ein zentrales Einsteckrohr (4), welches in Reaktorlängsrichtung angeordnet ist, einströmt, dieses von oben nach unten durchströmt und in einer internen Schlaufenbewegung außerhalb des Einsteckrohrs (4) erneut nach oben strömt,
- mit einem Wärmetauscher (6) im innenraum des Reaktors (1), durch den Kühlwasser strömt, und hierbei einen Teil der Reaktionswärme aufnimmt,
- mit Zuführung des Dinitrotoluols am oberen Endes des Reaktors (1) und Zuführung des Wasserstoffs am unteren Ende des Reaktors (1),
- und wobei zusätzlich zu dem im Innenraum des Reaktors (1) angeordneten Wärmetauscher (6) ein weiterer Wärmetauscher (W), in der externen Schlaufe eingesetzt wird, worin Wasser durch indirekten Wärmetausch mit der Reaktionsmischung die restliche Reaktionswärme aufnimmt,
**dadurch gekennzeichnet, dass**
die Reaktionswärme zur Gewinnung von Dampf mit einem Überdruck von mindestens 4 bar genutzt wird, indem die Hydrierung von Dinitrotoluol zu Toluyfendiamin bei einer Temperatur von größer oder gleich 180°C durchgeführt wird, und dass das Dinitrotoluol in die Gasphase oberhalb des Früssigkeitsspiegels in den Reaktor (1) zugeführt wird, oder dass die Treibstrahldüse (2) als Zweistrahldüse ausgebildet ist, und dass Dinitrotoluol über einen Ringspalt an der Außenwand der als Zweistrahldüse ausgebildeten Treibstrahldüse (2) zugeführt wird.

2. Verfahren nach Anspruch 1, **dadurch gekennzeichnet, dass** die Hydrierung von Dinitrotoluol zu Toluylendiamin bei einer Temperatur von größer oder gleich 185°C durchgeführt wird.

3. Verfahren nach Anspruch 1 oder 2, **dadurch gekennzeichnet, dass** der Katalysator als Aktivmasse ein Metall, ausgewählt aus der Gruppe Platin, Palladium, Rhodium und Ruthenium, zusammen mit einem oder mehreren weiteren Metallen, ausgewählt aus der Gruppe Nickel, Kobalt, Eisen, Zink, enthält, die auf einem inerten Träger aufgebracht ist.

4. Verfahren nach Anspruch 3, **dadurch gekennzeichnet, dass** die Aktivmasse des Katalysators Platin und Nickel oder Palladium, Nickel und Kobalt, oder Palladium, Nickel und Eisen, enthält.

5. Verfahren nach einem der Ansprüche 1 bis 4, **dadurch gekennzeichnet, dass** der im Reaktorinnenraum angeordnete Wärmetauscher (6) ein Fieldrohr-Wärmetauscher ist.

6. Verfahren nach einem der Ansprüche 1 bis 4, **dadurch gekennzeichnet, dass** der im Reaktorinnenraum angeordnete Wärmetauscher (6) ein Rohrschlangen-Wärmetauscher ist.

7. Verfahren nach einem der Ansprüche 1 bis 4, **dadurch gekennzeichnet, dass** der Wärmetauscher (6) ein Rohrbündel-Wärmetauscher ist.

8. Verfahren nach einem der Ansprüche 1 bis 7, **dadurch gekennzeichnet, dass** der in der externen Schlaufe (3) angeordnete Wärmetauscher (W) ein Rohrbündelwärmetauscher ist.

9. Verfahren nach Anspruch 8, **dadurch gekennzeichnet, dass** das Dinitrotoluol über einen Ringverteiler (7) zugeführt wird.

10. Verfahren nach einem der Ansprüche 1 bis 9, **dadurch gekennzeichnet, dass** sichergestellt wird, dass die Konzentration des Wasserstoffs im Reaktionsgemisch, das aus dem Reaktorsumpf in die externe Schlaufe (3) strömt 1 Vol.-%, bevorzugt 3 Vol.-%, nicht unterschreitet.

11. Verfahren nach Anspruch 10, **dadurch gekennzeichnet, dass** die Mindestkonzentration des Wasserstoffs im Reaktionsgemisch, das aus dem Reaktionssumpf in die externe Schlaufe (3) strömt sichergestellt wird, indem der Durchmesser des Reaktors (1) oder die Abströmgeschwindigkeit des Reaktionsgemisches aus dem Reaktorsumpf entsprechend ausgelegt werden.

12. Verfahren nach Anspruch 10, **dadurch gekennzeichnet, dass** die Mindestkonzentration des Wasserstoffs im Reaktionsgemisch, das aus dem Reaktorsumpf in die externe Schlaufe (3) strömt sichergestellt wird, indem in das Reaktionsgemisch das aus dem Reaktorsumpf in die externe Schlaufe (3) strömt, möglichst nahe am Reaktor (1) Wasserstoff zugeführt wird.

## Claims

1. A process for preparing tolylenediamine by hydrogenating dinitrotoluene with hydrogen in the presence of a suspended catalyst
- in a vertically upright reactor (1),
- at the upper end of which is arranged a motive jet nozzle (2) through which the reaction mixture drawn off from the reactor bottom, via an external loop (3), is sprayed into the upper region of the reactor (1) and then flows into a central inserted tube (4) which is arranged in the longitudinal direction of the reactor, flows through the latter from the top downward and flows upward again outside the inserted tube (4) in an internal loop motion,
- with a heat exchanger (6) in the interior of the reactor (1), through which cooling water flows, and absorbs some of the heat of reaction as it does so,
- with a feed for the dinitrotoluene at the upper end of the reactor (1) and a feed for the hydrogen at the lower end of the reactor (1),
- and wherein, in addition to the heat exchanger (6) arranged in the interior of the reactor (1), a further heat exchanger (W) is used in the external loop, in which water absorbs the rest of the heat of reaction by indirect heat exchange with the reaction mixture,
which comprises
utilizing the heat of reaction to raise steam with a pressure of at least 4 bar gauge by performing the hydrogenation of dinitrotoluene to tolylenediamine at a temperature of greater than or equal to 180°C, and the dinitrotoluene supplying to the reactor (1) into the gas phase above the liquid level, or the motive jet nozzle (2) configuring as a two-jet nozzle, and supplying dinitrotoluene to the motive jet nozzle (2) configured as a two-jet nozzle via an annular gap on the outer wall.

2. The process according to claim 1, wherein the hydrogenation of dinitrotoluene to tolylenediamine is performed at a temperature of greater than or equal to 185°C.

3. The process according to claim 1 or 2, wherein the catalyst comprises, as an active material, a metal selected from the group of platinum, palladium, rhodium and ruthenium, together with one or more further metals selected from the group of nickel, cobalt, iron, zinc, which are applied to an inert support.

4. The process according to claim 3, wherein the active material of the catalyst comprises platinum and nickel, or palladium, nickel and cobalt, or palladium, nickel and iron.

5. The process according to any one of claims 1 to 4, wherein the heat exchanger (6) arranged in the reactor interior is a Field tube heat exchanger.

6. The process according to any one of claims 1 to 4, wherein the heat exchanger (6) arranged in the reactor interior is a coiled tube heat exchanger.

7. The process according to any one of claims 1 to 4, wherein the heat exchanger (6) is a tube bundle heat exchanger.

8. The process according to any one of claims 1 to 7, wherein the heat exchanger (W) arranged in the external loop (3) is a tube bundle heat exchanger.

9. The process according to claim 8 wherein the dinitrotoluene is supplied by means of a ring distributor (7).

10. The process according to any one of claims 1 to 9, wherein it is ensured that the concentration of the hydrogen in the reaction mixture which flows out of the reactor bottom into the external loop (3) does not go below 1% by volume, preferably 3% by volume.

11. The process according to claim 10, wherein the minimum concentration of the hydrogen in the reaction mixture which flows out of the reaction bottoms into the external loop (3) is ensured by correspondingly designing the diameter of the reactor (1) or the flow rate of the reaction mixture out of the reactor bottom.

12. The process according to claim 10, wherein the minimum concentration of the hydrogen in the reaction mixture which flows out of the reactor bottom into the external loop (3) is ensured by supplying hydrogen as close as possible to the reactor (1) into the reaction mixture which flows out of the reactor bottom into the external loop (3).

## Revendications

1. Procédé pour la production de toluylènediamine par hydrogénation de dinitrotoluène à l'aide d'hydrogène en présence d'un catalyseur en suspension
- dans un réacteur (1) disposé verticalement,
- à l'extrémité supérieure duquel est placée une buse à jet de propulsion (2), par laquelle dans la zone supérieure du réacteur (1) est injecté via une boucle externe (3) le mélange réactionnel évacué du fond du réacteur, qui pénètre ensuite dans un tube inséré central (4), qui est disposé dans le sens longitudinal du réacteur, circule dans celui-ci de haut en bas et de nouveau circule vers le haut en un mouvement faisant une boucle interne à l'extérieur du tube inséré (4),
- avec à l'intérieur du réacteur (1) un échangeur de chaleur (6) dans lequel de l'eau de refroidissement circule et absorbe ainsi une partie de la chaleur de réaction,
- avec introduction du dinitrotoluène à l'extrémité supérieure du réacteur (1) et introduction de l'hydrogène à l'extrémité inférieure du réacteur (1),
- et en utilisant, en plus de l'échangeur de chaleur (6) placé à l'intérieur du réacteur (1), un autre échangeur de chaleur (W) dans la boucle externe, dans lequel de l'eau absorbe la chaleur de réaction restante par échange thermique indirect avec le mélange réactionnel,
**caractérisé en ce**
**qu'**on utilise la chaleur de réaction pour l'obtention de vapeur sous une surpression d'au moins 4 bars, en effectuant l'hydrogénation du dinitrotoluène en toluylènediamine à une température supérieure ou égale à 180 °C, et en ce qu'on introduit le dinitrotoluène dans la phase gazeuse au-dessus du niveau du liquide dans le réacteur (1), ou en ce que la buse à jet de propulsion (2) est constituée en tant que buse à double jet, et en ce qu'on introduit le dinitrotoluène par un espace annulaire au niveau de la paroi externe de la buse à jet de propulsion (2) constituée en tant que buse à double jet.

2. Procédé selon la revendication 1, **caractérisé en ce qu'**on effectue l'hydrogénation du dinitrotoluène en toluylènediamine à une température supérieure ou égale à 185 °C.

3. Procédé selon la revendication 1 ou 2, **caractérisé en ce que** le catalyseur contient en tant que matière active un métal, choisi dans le groupe constitué par le platine, le palladium, le rhodium et le ruthénium, conjointement avec un ou plusieurs autres métaux, choisis dans le groupe constitué par le nickel, le cobalt, le fer, le zinc, qui est appliquée sur un support inerte.

4. Procédé selon la revendication 3, **caractérisé en ce que** la matière active du catalyseur contient du platine et du nickel ou du palladium, du nickel et du cobalt, ou du palladium, du nickel et du fer.

5. Procédé selon l'une quelconque des revendications 1 à 4, **caractérisé en ce que** l'échangeur de chaleur (6) placé à l'intérieur du réacteur est un échangeur de chaleur à tubes Field.

6. Procédé selon l'une quelconque des revendications 1 à 4, **caractérisé en ce que** l'échangeur de chaleur (6) placé à l'intérieur du réacteur est un échangeur de chaleur à serpentin.

7. Procédé selon l'une quelconque des revendications 1 à 4, **caractérisé en ce que** l'échangeur de chaleur (6) est un échangeur de chaleur à faisceau de tubes.

8. Procédé selon l'une quelconque des revendications 1 à 7, **caractérisé en ce que** l'échangeur de chaleur (W) placé dans la boucle externe (3) est un échangeur de chaleur à faisceau de tubes.

9. Procédé selon la revendication 8, **caractérisé en ce qu'**on introduit le dinitrotoluène par un distributeur annulaire (7).

10. Procédé selon l'une quelconque des revendications 1 à 9, **caractérisé en ce qu'**il est assuré que la concentration de l'hydrogène dans le mélange réactionnel, qui circule dans la boucle externe (3) en sortant du fond du réacteur, n'est pas inférieure à 1 % en volume, de préférence 3 % en volume.

11. Procédé selon la revendication 10, **caractérisé en ce qu'**on assure la concentration minimale de l'hydrogène dans le mélange réactionnel, qui circule dans la boucle externe (3) en sortant du fond de la réaction, en concevant convenablement le diamètre du réacteur (1) ou la vitesse d'écoulement du mélange réactionnel sortant du fond du réacteur.

12. Procédé selon la revendication 10, **caractérisé en ce qu'**on assure la concentration minimale de l'hydrogène dans le mélange réactionnel, qui circule dans la boucle externe (3) en sortant du fond du réacteur, en introduisant le plus près possible du réacteur (1) l'hydrogène dans le mélange réactionnel qui circule dans la boucle externe (3) en sortant du fond du réacteur.
